Europäisches Patentamt

European Patent Office

Office européen des brevets

(19)

(11) **EP 1 627 665 A2**

(12) **DEMANDE DE BREVET EUROPEEN**

(43) Date de publication:
**22.02.2006 Bulletin 2006/08**

(51) Int Cl.:
***A61Q 5/06*** (2006.01)  ***A61K 8/04*** (2006.01)
***A61K 8/85*** (2006.01)

(21) Numéro de dépôt: **05291492.6**

(22) Date de dépôt: **11.07.2005**

(84) Etats contractants désignés:
**AT BE BG CH CY CZ DE DK EE ES FI FR GB GR
HU IE IS IT LI LT LU LV MC NL PL PT RO SE SI
SK TR**
Etats d'extension désignés:
**AL BA HR MK YU**

(30) Priorité: **29.07.2004  FR 0451706**

(71) Demandeur: **L'OREAL**
**75008 Paris (FR)**

(72) Inventeurs:
• **Bebot, Cécile**
  **92110 Clichy (FR)**
• **Laurent, Ludivine**
  **92400 Courbevoie (FR)**
• **Vrignaud, Sabine**
  **92110 Clichy (FR)**

(74) Mandataire: **Bourdeau, Françoise**
**L'OREAL - D.I.P.I.**
**25-29 Quai Aulagnier**
**92600 Asnières (FR)**

(54) **Polyester sulfonique linéaire comme agent de fixation des cheveux résistant à l'eau**

(57)      La présente demande a pour objet l'utilisation d'au moins un polyester sulfonique linéaire dispersible dans l'eau, comme agent de fixation des cheveux résistant à l'eau liquide.

**EP 1 627 665 A2**

**Description**

La présente demande a pour objet l'utilisation d'au moins un polyester sulfonique linéaire dispersible dans l'eau, comme agent de fixation des cheveux résistant à l'eau.

Les compositions cosmétiques pour la mise en forme et/ou le maintien de la coiffure peuvent être des compositions à pulvériser essentiellement constituées d'une solution le plus souvent alcoolique et d'un ou plusieurs composants, appelés composants fixants, qui sont généralement des résines polymères dont la fonction est de former des soudures entre les cheveux ou de gainer les cheveux. Ces composants fixants sont souvent formulés en mélange avec divers adjuvants cosmétiques.

Ces compositions cosmétiques sont généralement conditionnées soit dans un flacon pompe, soit dans un récipient aérosol approprié mis sous pression à l'aide d'un agent propulseur, le système aérosol contient alors d'une part une phase liquide (ou jus) et d'autre part d'un agent propulseur.

On peut également utiliser des compositions de coiffage sous forme de gels, de crèmes, de mousses.

Une fois appliquée sur les cheveux, les composés fixants doivent permettre la fixation de la chevelure.

Or les polymères couramment utilisés en tant qu'agents fixants dans les compositions de coiffage ne permettent pas de conserver la mise en forme de la coiffure lorsque celle-ci est mise au contact de l'eau liquide, pendant une durée prolongée telle que par exemple une mise en contact avec la pluie, la transpiration, ou lors des bains : bains de mer ou bains en piscine par exemple.

De manière surprenante est avantageuse, la demanderesse a découvert que l'utilisation d'au moins un polyester sulfonique linéaire dispersible dans l'eau, dans un milieu cosmétiquement acceptable, à une concentration comprise entre 5 et 40 % par rapport au poids total de la composition cosmétique, a pour effet de pour fixer et de mettre en forme les coiffures et aussi de conserver la mise en forme des coiffures lorsque celles-ci sont mise au contact de l'eau liquide pendant une durée prolongée.

Au sens de la présente demande, on nomme ce phénomène de « résistance à l'eau liquide ».

Par « durée prolongée » au sens de la présente demande, on entend une mise en contact avec l'eau d'une durée minimale d'une minute, de préférence dix minutes et de manière encore plus préférée 20 minutes.

On peut évaluer 1a conservation de la mise en forme par un test métrologique. Le protocole de test est le suivant

Protocole opératoire :

Sur mèche de cheveux naturels de 2.7g et de longueur 27 cm, appliquer 2 g de la formule à tester.

- Enrouler la mèche traitée autour d'un bigoudi de diamètre 1 cm, pour lui donner une forme.

- Laisser sécher le produit à l'air, puis défaire délicatement la mèche du bigoudi.

- Les mèches ainsi mises en forme sont ensuite immergées dans un bain d'eau salée (3% NaCl) de volume 8 litres à température ambiante, sous agitation magnétique à 100 tours/minute.

- Mesurer la longueur de la mèche au cours du temps afin d'évaluer le maintien de la mise en forme.

Mesures du maintien de la mise en forme :

$$\text{Maintien de la mise en forme en \% : } (L_i - L)/(L_i - L_0)*100$$

$L$ : Longueur de la mèche bouclée au temps t
$L_0$ : Longueur de la mèche bouclée après mise en forme et retrait du bigoudi
$L_i$ : Longueur de la mèche avant mise en forme sur bigoudi

De préférence, on retient les compositions permettant d'obtenir une conservation de la mise en forme supérieure à 70% pour un temps d'immersion de 10 minutes : on parle alors de résistance à l'eau. Avec les polyesters sulfoniques linéaires de l'invention, on obtient une conservation de la mise en forme de ce type.

Les compositions de l'invention permettent également d'obtenir un coiffage résistant à l'humidité de l'air.

Les compositions selon la présente invention permettent une bonne fixation et un bon maintien des cheveux c'est-à-dire un effet coiffant qui persiste tout au long de la journée, voire plusieurs jours, qui présente une bonne résistance à l'eau, notamment une bonne résistance aux bains répétés, ces compositions présentent aussi l'avantage de s'éliminer au shampooing.

Ces compositions permettent aussi de conférer de bonnes propriétés cosmétiques.

La présente invention a pour objet l'utilisation comme agent de fixation de la forme d'une chevelure résistant à l'eau liquide, d'au moins un polyester sulfonique linéaire dispersible dans l'eau, présentant une température de transition vitreuse comprise entre 50 et 100°C, à une concentration relative comprise entre 5 et 40 % en poids dans un milieu cosmétiquement acceptable.

La température de transition vitreuse (Tg) est mesurée par analyse enthalpique différentielle (DSC, *differential scanning calorimetry*) selon la norme ASTM D3418-97.

Avantageusement, le milieu cosmétiquement acceptable est exempt de corps gras choisis parmi les huiles minérales, végétales et les cires.

Plus avantageusement, le milieu cosmétiquement acceptable comprend entre 20 et 95 % en poids d'eau, de préférence entre 30 et 85%, plus préférentiellement entre 40 et 80%.

Plus avantageusement, le milieu cosmétiquement acceptable comprend entre 0 et 20 % en poids d'alcool en C1 à C4, de préférence entre 0 et 10 %, plus préférentiellement entre 1 et 6 %.

La composition cosmétique utilisée selon l'invention peut se présenter sous toutes les formes : lotions, sprays, mousses, gels, crèmes.

Préférentiellement, la viscosité de la composition cosmétique est comprise entre 1cps et $10^5$ cps, mesuré à 25°C et avec un taux de cisaillement de 1 $s^{-1}$.

D'autres objets, caractéristiques, aspects et avantages de l'invention apparaîtront encore plus clairement à la lecture de la description et des exemples qui suivent.

Par « composition cosmétique coiffante » au sens de la présente demande, on entend une composition pour la mise en forme ou le maintien de la coiffure.

La composition selon l'invention comprend un polyester sulfonique linéaire dispersible dans l'eau.

Par polyester sulfonique linéaire hydrodispersible, on entend tout polyester sulfonique présentant une aptitude à former une dispersion, c'est-à-dire un système biphasique où la première phase est formée de particules finement divisées et distribuées uniformément dans la seconde phase qui est la phase continue.

Par polyester sulfonique, on entend des copoylesters obtenus par polycondensation d'au moins un acide dicarboxylique ou d'un de ses esters, d'au moins un diol et d'au moins un composé difonctionnel sulfoaryldicarboxylique substitué sur le noyau aromatique par un groupe -$SO_3$M dans lequel M représente un atome d'hydrogène ou un ion métallique tel que $Na^+$, $Li^+$ ou $K^+$.

Les polyesters sulfoniques linéaires dispersibles dans l'eau présentent généralement une masse moléculaire moyenne en poids comprise entre environ 1 000 et 60 000, et de préférence de 4 000 à 20 000, telle que déterminée par chromatographie par perméation de gel (ou GPC).

La température de transition vitreuse (Tg )de ces polyesters sulfoniques est préférentiellement comprise dans l'intervalle allant de 50 °C à 100 °C, de préférence de 50 à 70 °C.

Ils sont décrits plus en détail dans les demandes de brevet US 3 734 874, US 3 779 993, US 4 119 680, US 4 300 580, US 4 973 656, US 5 660 816, US 5 662 893, et US 5 674 479.

Avantageusement, le polyester sulfonique linéaire dispersible dans l'eau est un polycondensat d'au moins un acide dicarboxylique ou d'un de ses esters, d'au moins un diol et d'au moins un composé difonctionnel sulfoaryldicarboxylique substitué sur le noyau aromatique par un groupe -$SO_3$M dans lequel M représente un atome d'hydrogène ou un ion métallique tel que $Na^+$, $Li^+$ ou $K^+$.

Plus avantageusement, le polyester sulfonique linéaire dispersible dans l'eau est obtenu à partir d'acide isophtalique, de sel de sodium de l'acide sulfoisophtalique, de diéthylèneglycol et de 1,4-cyclohexanemethanol.

Les polyesters sulfoniques utilisés de préférence dans l'invention comprennent au moins des motifs dérivés d'acide isophtalique, de sel d'acide sulfoaryle-dicarboxylique et de diéthylèneglycol, et plus particulièrement les polyesters sulfoniques utilisés dans l'invention sont obtenus à partir d'acide isophtalique, de sel de sodium de l'acide sulfoisophtalique, de diéthylèneglycol et de 1,4-cyclohexanemethanol.

A titre d'exemples de polyester sulfonique, on peut notamment citer ceux connus sous le nom INCI Diglycol/ CHDM/Isophtalates/SIP, et vendus sous la dénomination commerciale"Eastman AQ polymer" (par la société Eastman Chemical.

La concentration en polyester(s) sulfonique(s) linéaire(s) dispersible(s) dans l'eau utilisé(s) dans les compositions selon la présente invention est comprise entre 5 et 40 %, de préférence entre 5 et 30 % et de manière encore plus préférée entre 5 et 25% en poids par rapport au poids total de la composition.

La composition coiffante selon l'invention peut contenir en outre au moins un additif choisi parmi les agents tensio-actifs non-ioniques, anioniques, cationiques et amphotères, les polymères non-ioniques, anioniques, cationiques et amphotères additionnels, les céramides et pseudo-céramides, les vitamines et pro-vitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions selon la présente invention.

Ces additifs sont présents dans la composition selon l'invention en une quantité allant de 0 à 20 % en poids par rapport au poids total de la composition.

La composition de l'invention pourra se présenter sous la forme d'un spray aérosol ou non, d'une mousse, d'un gel, d'une pâte ou d'une crème.

Avantageusement, l'invention concerne l'utilisation d'au moins un polyester sulfonique linéaire avec au moins un tensio-actif non ionique pour la formulation d'une mousse de coiffage ainsi que l'utilisation d'au moins un polyester sulfonique linéaire pour l'obtention de compositions permettant une conservation de la mise en forme supérieure à 70% pour un temps d'immersion de 10 min dans un test de résistance à l'eau.

Les exemples suivants illustrent la présente invention et ne doivent être considérés en aucune manière comme limitant l'invention.

EXEMPLES

On prépare les formulations suivantes :

Spray non aérosol :

| Formulation A | en % de matière active |
|---|---|
| DIGLYCOL/CHDM/ISOPHTHALATES/SIP COPOLYMER (EASTMAN AQ 55 S - EASTMAN) | 10 |
| EAU DESIONISEE | QS 100 |
| Parfum et ingrédients | QSP |

Mousse de coiffage

| Formulation B | en % de matière active |
|---|---|
| DIGLYCOL/CHDM/ISOPHTHALATES/SIP COPOLYMER (EASTMAN AQ 55 S - EASTMAN) | 10 |
| Monolaurate de sorbitane oxyéthyléné (20 OE) | 0.5 |
| isobutane/propane (85/15) | 5 |
| EAU DESIONISEE | QS 100 |
| Parfum et ingrédients | QSP |

Spray aérosol

| Formulation C | en % de matière active |
|---|---|
| DIGLYCOL/CHDM/ISOPHTHALATES/SIP COPOLYMER (EASTMAN AQ 55 S - EASTMAN) | 10 |
| DME | 40 |
| EAU DESIONISEE | QS 100 |
| Parfum et ingrédients | QSP |

Mesures du maintien de la mise en forme :

| Temps d'immersion | A | B | C |
|---|---|---|---|
| 0 | 100 | 100 | 100 |
| 25 secondes | 100 | 100 | 100 |
| 30 secondes | 100 | 100 | 100 |
| 35 secondes | 100 | 100 | 100 |
| 40 secondes | 100 | 100 | 100 |
| 10 minutes | 100 | 100 | 100 |

**Revendications**

1. Utilisation comme agent de fixation de la forme d'une chevelure résistant à l'eau liquide, d'au moins un polyester sulfonique linéaire dispersible dans l'eau, présentant une température de transition vitreuse comprise entre 50 et 100°C, à une concentration relative comprise entre 5 et 40 % en poids dans un milieu cosmétiquement acceptable.

2. Utilisation cosmétique selon la revendication 1 telle que le polyester sulfonique linéaire dispersible dans l'eau est un polycondensat d'au moins un acide dicarboxylique ou d'un de ses esters, d'au moins un diol et d'au moins un composé difonctionnel sulfoaryldicarboxylique substitué sur le noyau aromatique par un groupe -$SO_3M$ dans lequel M représente un atome d'hydrogène ou un ion métallique tel que $Na^+$, $Li^+$ ou $K^+$.

3. Utilisation cosmétique selon la revendication 1 ou 2 telle que le polyester sulfonique linéaire dispersible dans l'eau est obtenu à partir d'acide isophtalique, de sel de sodium de l'acide sulfoisophtalique, de diéthylèneglycol et de 1,4-cyclohexaneméthanol.

4. Utilisation cosmétique selon l'une des revendications 1 à 3 telle que la température de transition vitreuse (Tg) de ces polyesters sulfoniques est préférentiellement comprise dans l'intervalle allant de 50 °C à 100 °C, de préférence de 50 à 70 °C.

5. Utilisation cosmétique selon l'une des revendications 1 à 4 telle que la concentration en polyester(s) sulfonique(s) linéaire(s) dispersible(s) dans l'eau est comprise entre 5 et 40 %, de préférence entre 5 et 30 % et de manière encore plus préférée entre 5 et 25 % en poids par rapport au poids total de la composition.

6. Utilisation cosmétique selon l'une des revendications précédentes telle qu'elle contient au moins un additif choisi parmi les agents tensio-actifs non-ioniques, anioniques, cationiques et amphotères, les polymères non-ioniques, anioniques, cationiques et amphotères additionnels, les céramides et pseudo-céramides, les vitamines et pro-vitamines dont le panthénol, les filtres solaires hydrosolubles et liposolubles, siliconés ou non siliconés, les charges et particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

7. Utilisation d'au moins un polyester sulfonique linéaire selon l'une quelconque des revendications précédentes avec au moins un tensio-actif non ionique pour la formulation d'une mousse de coiffage.

8. Utilisation d'au moins un polyester sulfonique linéaire selon l'une quelconque des revendications précédentes, pour l'obtention de compositions permettant une conservation de la mise en forme supérieure à 70% pour un temps d'immersion de 10 min dans un test de résistance à l'eau .